(19) Europäisches Patentamt
European Patent Office
Office européen des brevets

(11) **EP 3 440 984 B1**

(12) **EUROPÄISCHE PATENTSCHRIFT**

(45) Veröffentlichungstag und Bekanntmachung des Hinweises auf die Patenterteilung:
**29.12.2021 Patentblatt 2021/52**

(51) Int Cl.:
**A47L 15/00** *(2006.01)*    **A61L 2/00** *(2006.01)*

(21) Anmeldenummer: **18187285.4**

(22) Anmeldetag: **03.08.2018**

(54) **VERFAHREN ZUR STEUERUNG DER DAUER EINER TROCKNUNGSPHASE EINES REINIGUNGSVERFAHRENS UND DAFÜR GEEIGNETES GERÄT**

METHOD OF CONTROLLING THE DURATION OF DRYING PHASE OF A CLEANING PROCESS AND DEVICE FOR SAME

PROCÉDÉ DE COMMANDE DE LA DURÉE D'UNE PHASE DE SÉCHAGE D'UN PROCÉDÉ DE NETTOYAGE ET APPAREIL DE NETTOYAGE APPROPRIÉ ASSOCIÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.08.2017 EP 17185763**

(43) Veröffentlichungstag der Anmeldung:
**13.02.2019 Patentblatt 2019/07**

(73) Patentinhaber: **MELAG Medizintechnik GmbH & Co. KG**
**10829 Berlin (DE)**

(72) Erfinder:
• **HALBICH, Johannes**
  **10405 Berlin (DE)**
• **LITZBA, Guido**
  **14513 Teltow (DE)**
• **MÖLLER, Janis**
  **22301 Hamburg (DE)**

• **PEISE, Oliver**
  **12557 Berlin (DE)**

(74) Vertreter: **Maikowski & Ninnemann Patentanwälte Partnerschaft mbB**
**Postfach 15 09 20**
**10671 Berlin (DE)**

(56) Entgegenhaltungen:
**WO-A1-2004/019750      CN-A- 105 105 694**
**DE-A1-102011 083 179   JP-A- H01 181 832**
**JP-A- 2011 200 392**

• **NADINA BAGHINA ET AL: "Predictive control of a domestic freezer for real-time demand response applications", INNOVATIVE SMART GRID TECHNOLOGIES (ISGT EUROPE), 2012 3RD IEEE PES INTERNATIONAL CONFERENCE AND EXHIBITION ON, IEEE, 14. Oktober 2012 (2012-10-14), Seiten 1-8, XP032332811, DOI: 10.1109/ISGTEUROPE.2012.6465809 ISBN: 978-1-4673-2595-0**

EP 3 440 984 B1

**Beschreibung**

Die vorliegende Erfindung betrifft ein Verfahren zur Steuerung der Dauer einer Trocknungsphase eines Verfahrens zur Reinigung und optionalen Desinfektion von Reinigungsgut gemäß dem Oberbegriff des Anspruchs 1 sowie ein Gerät, das im Betrieb ein solches Verfahren umsetzt, gemäß dem Oberbegriff des Anspruchs 8. Reinigungsgeräte oder Reinigungs- und Desinfektionsgeräte enthalten häufig ein integriertes Heizgebläse zur aktiven Trocknung der Beladung, da diese im Anschluss an den vorangegangenen Reinigungs- oder Desinfektionszyklus mit Wassertropfen benetzt ist. Für die aktive Trocknung wird Luft von einem Gebläse aus der Geräteumgebung angesaugt, mit Hilfe einer Heizung erhitzt und in das Innere einer Waschkammer des Geräts geleitet. Die auf dem Reinigungsgut (der sogenannten Beladung) befindlichen Tropfen verdunsten, und die Trocknungsluft reichert sich mit Feuchtigkeit an.

Am Ende der aktiven Trocknung ist die Wassermenge auf der Beladung gegenüber dem anfänglich vorliegenden Zustand reduziert. Allerdings verbleibt immer eine gewisse Restfeuchte auf der Beladung. Das Ausmaß der Restfeuchte ist von verschiedenen Faktoren abhängig, wie der Art und der Anzahl der zu trocknenden Beladungsgüter. Diese Faktoren erschweren es, ein für den Anwender reproduzierbares, zufriedenstellendes Trocknungsergebnis bei minimaler Trocknungszeit zu erzielen.

Aus dem Stand der Technik sind bereits einige Lösungen bekannt, die Trocknungszeit unter Berücksichtigung des tatsächlichen Feuchtigkeitsgrads der Beladung anzupassen.

Beispielsweise beschreibt das europäische Patent EP 1 689 280 B1 einen Geschirrspüler mit einem Feuchtigkeitssensor, der die Feuchtigkeit zumindest eines Teils der im Geschirrspüler vorhandenen Luft ermittelt. Der Trocknungsvorgang wird dann in Abhängigkeit von aktuell für die Feuchtigkeit ermittelten Messwerten des Feuchtigkeitssensors über eine elektronische Steuerung geregelt, damit die eingesetzten Wärmeerzeugungsmittel nur die erforderliche Wärmemenge produzieren.

Aus der DE 10 2008 043 176 A1 ist ein Trockner mit einem Zuluftkanal und einem Abluftkanal bekannt, die mittels eines Mechanismus über einen Umluftkanal zur Einstellung eines Anteils an Um- und Abluft im Trockner zusammengeschaltet werden können. Auf diese Weise lassen sich die Temperatur und die Feuchtigkeit in einem Innenraum des Trockners regeln. Dabei ist aus jener deutschen Patentanmeldung auch bekannt, einen Feuchtigkeitssensor zur Bestimmung der Feuchtigkeit der Luft des Raumes, in dem der Trockner aufgestellt ist, einzusetzen.

Aus der DE 10 2013 106 775 A1 ist ein Verfahren zum Trocknen von Spülgut in einem Geschirrspüler bekannt, bei der ein Differenzwert ermittelt wird, der einendem ein Temperaturunterschied zwischen der Außenluft und der Luft in der Behandlungskammer des Geschirrspülers ermittelt wird. Auf der Grundlage dieses Temperaturunterschieds wird dann das Trocknungsverfahren gesteuert.

Aus der EP 0 953 315 A1 ist ein Verfahren zum Trocknen von Spülgut in einer Geschirrspülmaschine bekannt, bei dem die Luftfeuchtigkeit innerhalb des Spülbehälters überwacht wird, um eine Tür des Geschirrspülers automatisch zu öffnen, wenn ein Grenzwert der relativen Luftfeuchtigkeit erreicht wird. Dabei wird in jener europäischen Patentanmeldung auch angedacht, die Luftfeuchtigkeit des Aufstellortes der Geschirrspülmaschine zu erfassen, um einen Programmschritt des Trocknungsgangs zu beeinflussen. Es wird allerdings nicht erläutert, wie eine derartige Beeinflussung konkret erfolgen könnte.

Die WO 2004/019750 A1 beschreibt ein Verfahren zur Trocknung erwärmter Güter in einer Haushaltsmaschine. Zu Beginn des Trocknungsverfahrens wird dabei ein einziger Wert der Feuchtigkeit der Zuluft bestimmt. Dazu wird derselbe Feuchtigkeitssensor verwendet, der im Laufe des Trocknungsverfahrens auch die Feuchtigkeit der Abluft bestimmt. Eine zusätzliche Temperaturbestimmung ist gemäß der Lehre dieser internationalen Patentanmeldung nicht vorgesehen. Es ist auch nicht vorgesehen, die Feuchtigkeit anhand absoluter Feuchtigkeitswerte zu bestimmen.

Aus der CN 105105694 A1 ist ein Verfahren zur Trocknung von Reinigungsgut in einer Geschirrspülmaschine bekannt, bei dem ebenfalls zu Beginn des Verfahrens der Feuchtigkeitsgehalt der Umgebungsluft durch eine einzige Messung bestimmt wird. Diese Feuchtigkeit wird dann als zu erreichende Zielfeuchtigkeit vorgegeben. Mithin zielt jene chinesische Patentanmeldung darauf ab, die Feuchtigkeit im Inneren der Geschirrspülmaschine auf den am Anfang bestimmten Zielfeuchtigkeitswert einzustellen.

Aus der JP 2011-200392 A ist ein Trocknungsverfahren bekannt, bei dem Zuluft zunächst in einem Entfeuchter vorgekühlt wird. Dadurch kondensiert Wasser, wodurch die Luftfeuchtigkeit der Zuluft sinkt. Anschließend wird die Zuluft wieder erwärmt und dann als getrocknete und erwärmte Luft zum Trocknen von Geschirr eingesetzt. Für die Überwachung der Lufttemperatur und des Erfolgs der Entfeuchtung werden gemäß der Lehre dieser japanischen Patentanmeldung zwei Temperatursensoren und zwei Feuchtigkeitssensoren eingesetzt. Diese Sensoren befinden sich alle im Lufteinlassbereich des Geräts. Die aus der Waschkammer austretende Abluft wird nicht in Bezug auf ihre Temperatur oder ihren Feuchtigkeitsgehalt überwacht.

Die DE 10 2011 083 179 A1 beschreibt eine Transportspülmaschine mit einer Abluftanlage, über die Abluft aus einer Behandlungszone abgeführt und einer Kondensationseinrichtung zugeführt werden kann. In der Kondensationseinrichtung wird der Abluft durch Kondensation Feuchtigkeit entzogen. Die Leistung der Kondensationseinrichtung ist dabei regelbar, um die Temperatur und den absoluten Feuchtigkeit der Abluft am Luftauslass der Abluftanlage einzustellen.

Der vorliegenden Erfindung liegt die Aufgabe zugrunde, ein verbessertes Verfahren zur Steuerung der Dauer einer Trocknungsphase eines Verfahrens zur Reinigung und optionalen Desinfektion von Reinigungsgut anzugeben. Dabei sollen die Dauer der Trocknungsphase in Abhängigkeit der Art und der Menge des zu trocknenden Spülgutes auf das notwendige Minimum reduziert und ein stets reproduzierbares Trocknungsergebnis erzielt werden. Der vorliegenden Erfindung liegt ferner die Aufgabe zugrunde, eine Vorrichtung anzugeben, die ein derartiges Verfahren implementiert.

Diese Aufgabe wird durch ein Verfahren zur Steuerung der Dauer einer Trocknungsphase eines Verfahrens zur Reinigung und optionalen Desinfektion von Reinigungsgut mit den Merkmalen des Anspruchs 1 gelöst. Die Trocknungsphase kann die Trocknungsphase eines reinen Reinigungsverfahrens oder aber eines Reinigungs- und Desinfektionsverfahrens sein. Derartige Verfahren werden typischerweise in Reinigungsgeräten wie etwa Geschirrspülern oder im Reinigungs- und Desinfektionsgeräten durchgeführt. Sie dienen zur Reinigung und optionalen Desinfektion von Reinigungsgut wie etwa medizinischen oder zahnmedizinischen Instrumenten und Geräten.

Das erfindungsgemäß beanspruchte Verfahren zeichnet sich durch die nachfolgend erläuterten Schritte aus. In einem Verfahrensschritt wird eine Abluftfeuchtigkeit zu mindestens zwei voneinander verschiedenen Zeitpunkten $t_i$ unter Verwendung eines ersten Feuchtigkeitssensors gemessen. Die Abluftfeuchtigkeit gibt dabei eine Feuchtigkeit von Abluft an, welche aus einer Reinigungskammer, in der das zuvor gereinigte und optional auch desinfizierte Reinigungsgut getrocknet wird, ausströmt.

In einem weiteren Verfahrensschritt wird die der Temperatur von Abluft, welche aus einer Reinigungskammer, in der das Reinigungsgut getrocknet wird, ausströmt, mittels eines ersten Temperatursensors gemessen. Da die Temperatur zusätzlich zu der Feuchtigkeit bekannt ist, kann besonders einfach aus einem erhaltenen relativen Feuchtigkeitswert ein absoluter Feuchtigkeitswert berechnet werden, und umgekehrt. Durch die zusätzliche Temperaturinformation geben die mindestens zwei Werte für die Abluftfeuchtigkeit folglich jeweils eine absolute Feuchtigkeit der Abluft an.

In einem weiteren Verfahrensschritt wird eine Zuluftfeuchtigkeit zu denselben Zeitpunkten $t_i$ unter Verwendung eines zweiten Feuchtigkeitssensors gemessen. Die Zuluftfeuchtigkeit gibt eine Feuchtigkeit von Zuluft an, die aus einer Umgebung der Reinigungskammer (insbesondere aus einer Umgebung des Reinigungsgeräts oder des Reinigungs- und Desinfektionsgeräts, in dem das Verfahren durchgeführt wird) angesaugt wird. Die Zuluft dient zum Trocknen des Reinigungsguts in der Reinigungskammer.

Zusätzlich wird die Temperatur der Zuluft, welche zumindest teilweise aus einer Umgebung der Reinigungskammer angesaugt wird und zum Trocknen des Reinigungsguts verwendet wird, mittels eines zweiten Temperatursensors gemessen. Durch die zusätzliche Temperaturinformation geben auch die mindestens 2 Werte, die für die Zuluftfeuchtigkeit erhalten wurden, jeweils eine absolute Feuchtigkeit der Zuluft an.

Auf der Grundlage der mindestens zwei Werte der absoluten Feuchtigkeit der Abluft und der mindestens zwei Werte der absoluten Feuchtigkeit der Zuluft wird dann eine Zeitdauer berechnet, die voraussichtlich erforderlich ist, damit eine Differenz zwischen der absoluten Feuchtigkeit der Abluft und der absoluten Feuchtigkeit der Zuluft (also eine Differenz zwischen der Abluftfeuchtigkeit und der Zuluftfeuchtigkeit) einen vorgebbaren Schwellenwert unterschreitet.

Wenn die Zeitdauer verstrichen ist, wird die Trocknungsphase beendet. Denn dann ist das vorgebbare Trocknungskriterium (nämlich das Erreichen eines bestimmten Schwellenwerts) erfüllt, sodass das Reinigungsgut den gewünschten Trocknungsgrad erreicht hat.

Bei dem Schwellenwert kann es sich um einen festen, invariablen Schwellenwert handeln. Alternativ kann der Schwellenwert auch aus einem vordefinierten Bereich oder einer vordefinierten Gruppe möglicher Schwellenwerte ausgewählt werden.

Die Abluftfeuchtigkeit und die Zuluftfeuchtigkeit geben die absolute Feuchtigkeit (auch als absolute Feuchte bezeichnet) der Abluft bzw. der Zuluft an und werden in dieser Form nachfolgend für die Berechnung der Differenz verwendet.

In einer Variante ist der Schwellenwert aus einem Bereich von 0,1 bis 20 g/m$^3$, insbesondere von 0,2 bis 15 g/m$^3$, insbesondere von 0,3 bis 14 g/m$^3$, insbesondere von 0,4 bis 13 g/m$^3$, insbesondere von 0,5 bis 12 g/m$^3$, insbesondere von 0,6 bis 11 g/m$^3$, insbesondere von 0,7 bis 10 g/m$^3$, insbesondere von 0,8 bis 9 g/m$^3$, insbesondere von 0,9 bis 8 g/m$^3$, insbesondere von 1 bis 7 g/m$^3$, insbesondere von 2 bis 6 g/m$^3$, insbesondere von 3 bis 5 g/m$^3$ absoluter Feuchtigkeit ausgewählt. Dann gibt der Schwellenwert einen Wasserdampfgehalt von 0,1 bis 20 g pro Kubikmeter Luft bzw. einen entsprechenden Wasserdampfgehalt in einem der anderen vorgenannten Intervalle an. Ein aus dem Bereich von 0,1 bis 10 g/m$^3$ ausgewählter Schwellenwert eignet sich insbesondere für Reinigungskammern mit einem Nutzvolumen von bis zu 50, insbesondere bis zu 100 Litern. Ein aus dem Bereich von 0,1 bis 20 g/m$^3$ ausgewählter Schwellenwert eignet sich insbesondere für Reinigungskammern mit einem Nutzvolumen von mehr als 50, insbesondere mehr als 100 und bis zu 200 Litern. Ein besonders geeigneter Bereich zur Auswahl des Schwellenwertes ist der Bereich von 1 bis 5 g/m$^3$. Alternativ kann der Schwellenwert auch aus Werten relativer Feuchtigkeit ausgewählt werden, die den vorgenannten Werten der absoluten Feuchtigkeit entsprechen. Die relative Feuchtigkeit hängt dabei von der Temperatur der Abluft bzw. der Temperatur der Zuluft ab.

Wenn die Differenz zwischen der Abluftfeuchtigkeit und der Zuluftfeuchtigkeit kleiner als ein Wert ist, der innerhalb des vorgenannten Bereiches liegt, ist das Reinigungsgut hinreichend trocken, sodass eine weitere Trocknung nicht erforderlich ist. Diese würde auch wegen der geringen Differenz zwischen der Feuchtigkeit der Zuluft und der Feuchtigkeit der Abluft kaum für eine verbesserte Trocknung sorgen, gleichwohl aber eine Verlängerung der gesamten Trocknungsphase und eine Erhöhung der für die Trocknungsphase erforderlichen Energie zur Folge haben.

Die Differenz zwischen der Abluftfeuchtigkeit und der Zuluftfeuchtigkeit wird anhand von Messwerten der Abluftfeuchtigkeit und der Zuluftfeuchtigkeit berechnet. Dazu wird regelmäßig die Abluftfeuchtigkeit zu einem Zeitpunkt $t_i$ gemessen. Die Zuluftfeuchtigkeit wird zum selben Zeitpunkt $t_i$ ebenfalls gemessen. Zur Berechnung der Differenz kann jedoch auch alternativ auf einen älteren Wert der Zuluftfeuchtigkeit, der beispielsweise zu Beginn der Trocknungsphase ermittelt wurde, zurückgegriffen werden. Denn die Zuluftfeuchtigkeit ändert sich im Vergleich zur Abluftfeuchtigkeit während der Trocknungsphase nur wenig, sofern das Verfahren in einem Gerät durchgeführt wird, das in einem gut belüfteten Raum aufgestellt ist.

In einer Variante wird zur Berechnung der Zeitdauer eine Differenz zwischen der zu einem bestimmten Zeitpunkt $t_i$ gemessenen Abluftfeuchtigkeit und der zum selben Zeitpunkt $t_i$ gemessenen Zuluftfeuchtigkeit gebildet. Dies ist die genaueste Variante, um die Zeitdauer zu berechnen, da sie die tatsächlichen Feuchtigkeitsverhältnisse in Abluft und Zuluft am besten berücksichtigt.

Da grundsätzlich jederzeit Änderungen in der Feuchtigkeit der Zuluft eintreten können (insbesondere dann, wenn das Verfahren in einem kleineren, schlecht belüfteten Raum durchgeführt wird und sich die Feuchtigkeit der Zuluft dann durch die aus dem Gerät abgeführte Abluft erhöht), kann sich auch ein zuvor erwarteter Trocknungserfolg während der Durchführung des Verfahrens zu einem bestimmten Zeitpunkt noch nicht eingestellt haben. Andererseits ist es auch möglich, dass sich die Feuchtigkeit der Zuluft verringert, weshalb ein erwarteter Trocknungserfolg schneller als ursprünglich gedacht erreicht werden kann. Mit anderen Worten ausgedrückt, kann sich die Zeitdauer, die voraussichtlich erforderlich ist, damit eine Differenz zwischen der Abluftfeuchtigkeit und der Zuluftfeuchtigkeit den Schwellenwert unterschreitet, im Laufe des Verfahrens verändern. In einer Variante ist es daher vorgesehen, dass die Zeitdauer zwischen Schritt d) und Schritt e) erneut berechnet wird. Dazu wird ein weiteres Wertepaar der Abluftfeuchtigkeit und der Zuluftfeuchtigkeit verwendet, das zu einem weiteren (späteren) Zeitpunkt $t_i$ gemessen wurde. Die derart neu berechnete Zeitdauer ersetzt dann die zuvor berechnete Zeitdauer.

Die Messzeitpunkte $t_i$ beziehen sich auf eine Zeitskala, die an Zeitpunkt $t = 0$ beginnt. Der Zeitpunkt $t$ = 0 muss nicht notwendigerweise der unmittelbare Beginn der Trocknungsphase sein. Vielmehr ist es in einer Variante vorgesehen, den Zeitpunkt $t = 0$ auf einen Zeitpunkt zu setzen, der nach dem tatsächlichen Beginn der Trocknungsphase liegt. Denn es hat sich gezeigt, dass zu Beginn der Trocknungsphase aufgrund eines sich noch nicht eingestellten Gleichgewichts sowie aufgrund von Sensorartefakten nicht in jedem Fall verlässliche Messwerte zur Bestimmung der initialen Abluftfeuchtigkeit erhalten werden können. Daher kann der Zeitpunkt $t = 0$ in einer Variante einer Zeit entsprechen, die in einem Bereich von 1 Minute bis 10 Minuten, insbesondere 1,5 Minuten bis 9 Minuten, insbesondere 2 Minuten bis 8 Minuten, insbesondere 2,5 Minuten bis 7 Minuten, insbesondere 3 Minuten bis 6 Minuten, insbesondere 3,5 Minuten bis 5 Minuten, insbesondere 4 Minuten bis 4,5 Minuten und ganz besonders rund 3 Minuten (beispielsweise 2,5 Minuten bis 3,5 Minuten) nach dem tatsächlichen Start der Trocknungsphase liegt.

In einer Variante wird die Zeitdauer, die zum Erreichen des Schwellenwerts (voraussichtlich) erforderlich ist, angezeigt, um einem Benutzer Informationen darüber zu geben, wie lange die Trocknungsphase dauern wird.

In einer Variante wird die Zeitdauer gemäß folgender Formel berechnet:

$$t_{ges} = log_a \frac{F_S}{b}$$

wobei

$$a = \left(\frac{\Delta F_{t_2}}{\Delta F_{t_1}}\right)^{\frac{1}{t_2 - t_1}}$$

$$b = \frac{\Delta F_{t_1}}{a^{t_1}}$$

$t_{ges}$  die Zeitdauer,
$F_S$  der Schwellenwert der Differenz zwischen der Abluftfeuchtigkeit und der Zuluftfeuchtigkeit,
$t_1$  ein erster Messzeitpunkt während des Trocknungsprozesses,
$t_2$  ein nach dem ersten Messzeitpunkt liegender zweiter Messzeitpunkt während des Trocknungsprozesses,
$\Delta F_{t1}$  die Differenz zwischen der Abluftfeuchtigkeit und der Zuluftfeuchtigkeit zum Zeitpunkt $t_i = t_1$ und
$\Delta F_{t2}$  die Differenz zwischen der Abluftfeuchtigkeit und der Zuluftfeuchtigkeit zum Zeitpunkt $t_i = t_2$ ist.

In einer Variante wird der Schwellenwert in Abhängigkeit des Faktors a und/oder des Faktors b vorgegeben. Dies kann unabhängig davon erfolgen, ob die Zeitdauer gemäß der vorstehend erläuterten Formel berechnet wird oder nicht. Die Faktoren a und/oder b können also unabhängig von der vorstehend erläuterten Formel berechnet und zur Bestimmung des Schwellenwerts herangezogen werden.

In einer Variante werden eine Trocknungstemperatur, die während der Trocknungsphase in der Reinigungskammer herrscht, und/oder eine Drehzahl eines Gebläses, mit dem die Zuluft in die Reinigungskammer gefördert wird, in Abhängigkeit des Faktors a und/oder des Faktors b eingestellt. Auch dies kann unabhängig davon erfolgen, ob die Zeitdauer gemäß der vorstehend erläuterten Formel berechnet wird oder nicht. Die Faktoren a und/oder b können also unabhängig von der vorstehend erläuterten Formel berechnet und zur Einstellung der Trocknungstemperatur und/oder der Gebläsedrehzahl herangezogen werden.

In einer Variante sind die zur Berechnung der Zeitdauer herangezogenen Werte der Abluftfeuchtigkeit und/oder der Zuluftfeuchtigkeit Mittelwerte aus gemessenen Werten der Abluftfeuchtigkeit und/oder der Zuluftfeuchtigkeit. Dadurch können kurzzeitige Schwankungen der Abluftfeuchtigkeit und/oder der Zuluftfeuchtigkeit besonders vorteilhaft ausgeglichen werden. Als theoretischer Messzeitpunkt, auf den sich die derart gebildeten Mittelwerte beziehen, wird dann insbesondere ebenfalls ein Mittelwert der tatsächlich gemessenen und für die Mittelwertbildung berücksichtigten Messzeitpunkte verwendet.

Messergebnisse von Feuchtigkeitssensoren weisen häufig eine Abhängigkeit von anderen Umgebungseinflüssen, wie beispielsweise der Temperatur, auf. Man spricht in diesem Zusammenhang auch von einem Temperaturgang der Feuchtigkeitssensoren. In einer Variante wird ein Korrekturfaktor für einen Messwert der Abluftfeuchtigkeit und/oder der Zuluftfeuchtigkeit bestimmt. Dieser Korrekturfaktor wird dann auf den entsprechenden Messwert angewandt. Es resultiert ein korrigierter Messwert der Abluftfeuchtigkeit und/oder der Zuluftfeuchtigkeit, der genauer als der ursprüngliche Messwert ist. Der Korrekturfaktor kann dabei beispielsweise den Temperaturgang des zur Messung der Abluftfeuchtigkeit und/oder der Zuluftfeuchtigkeit eingesetzten Sensors kompensieren. Es können auch mehrere Korrekturfaktoren für unterschiedliche Umwelteinflüsse verwendet werden, um insgesamt genauere Messergebnisse für die Abluftfeuchtigkeit und/oder die Zuluftfeuchtigkeit zu erhalten. Dies ist insbesondere dann von Vorteil, wenn die Feuchtigkeitsdifferenz zwischen der Abluftfeuchtigkeit und der Zuluftfeuchtigkeit gering ist. Denn je geringer diese Differenz ist, desto größer wird der Einfluss von Messfehlern bei der Messung der Abluftfeuchtigkeit und/oder der Zuluftfeuchtigkeit mittels eines Sensors. Gerade bei einer geringen Restfeuchte (Abluftfeuchtigkeit minus Zuluftfeuchtigkeit) stoßen Feuchtigkeitssensoren an ihre physikalischen Grenzen, sodass entsprechende Messwerte nur noch bedingt zur Durchführung des vorliegend beanspruchten Verfahrens geeignet sind.

Die vorliegende Erfindung betrifft auch eine Vorrichtung zur Reinigung und optionalen Desinfektion von Reinigungsgut mit den nachfolgend erläuterten Merkmalen. Bei einer solchen Vorrichtung kann es sich beispielsweise um ein Reinigungsgerät wie einen Geschirrspüler oder um ein Reinigungs- und Desinfektionsgerät handeln, das beispielsweise zur Reinigung und Desinfektion von ärztlichen oder zahnärztlichen Instrumenten und Geräten verwendet wird. Die Vorrichtung weist eine Reinigungskammer zur Aufnahme von Reinigungsgut auf. Darüber hinaus sind ein erster Feuchtigkeitssensor, ein zweiter Feuchtigkeitssensor und ein Steuergerät vorgesehen.

Die Vorrichtung weist ferner einen ersten Temperatursensor auf, der zur Messung einer Temperatur von aus der Reinigungskammer austretender Abluft dient. Außerdem weist die Vorrichtung einen zweiten Temperatursensor auf, der zur Messung einer Temperatur von zumindest teilweise aus einer Umgebung der Reinigungskammer angesaugter Zuluft dient. Der erste Feuchtigkeitssensor dient zur Messung eines (relativen) Feuchtigkeitswerts einer Abluftfeuchtigkeit. Zusammen mit der gemessenen Temperatur der Abluft lässt sich so die absolute Feuchtigkeit der Abluft berechnen. Der zweite Feuchtigkeitssensor dient zur Messung eines (relativen) Feuchtigkeitswerts einer Zuluftfeuchtigkeit von Zuluft, die aus einer Umgebung der Reinigungskammer angesaugt wird. Zusammen mit der gemessenen Temperatur der Zuluft lässt sich so die absolute Feuchtigkeit der Zuluft berechnen.

Die Vorrichtung zeichnet sich erfindungsgemäß dadurch aus, dass das Steuergerät spezifisch dafür hergerichtet ist, dass es im Betrieb der Vorrichtung ein Verfahren gemäß den obigen Erläuterungen ausführt. So berechnet das Steuergerät auf der Grundlage der absoluten Feuchtigkeit der Abluft und der absoluten Feuchtigkeit der Zuluft eine Zeitdauer, die voraussichtlich erforderlich ist, damit eine Differenz zwischen der absoluten Feuchtigkeit der Abluft und der absoluten Feuchtigkeit der Zuluft einen vorgebbaren Schwellenwert unterschreitet. Das Steuergerät ist ferner spezifisch dafür hergerichtet, eine Trocknungsphase eines Verfahrens zur Reinigung und optionalen Desinfektion von in der Reinigungskammer aufgenommenem Reinigungsgut zu beenden, wenn die Zeitdauer verstrichen ist.

Bei dem ersten Feuchtigkeitssensor und/oder dem zweiten Feuchtigkeitssensor handelt es sich in einer Variante um ein kapazitives Hygrometer, ein Absorptionshygrometer, ein Psychrometer, ein Taupunktspiegelhygrometer, ein optisches Hygrometer, ein resistives Hygrometer oder ein coulometrisches Hygrometer. Dabei können der erste Feuchtigkeitssensor und der zweite Feuchtigkeitssensor unabhängig voneinander durch einen der vorgenannten Feuchtigkeitssensortypen realisiert werden. In einer Variante sind der erste Feuchtigkeitssensor und der zweite Feuchtigkeitssensor baugleich.

In einer weiteren Variante weist die Vorrichtung einen ersten Drucksensor auf, der zur Messung eines Luftdrucks innerhalb der Reinigungskammer oder von aus der Reinigungskammer austretender Abluft dient, und einen zweiten Drucksensor, der zur Messung eines Luftdrucks von aus einer Umgebung der Reinigungskammer angesaugter Zuluft dient. Dann lassen sich weitere Messdaten generieren, die beispielsweise zur Erhöhung der Genauigkeit der Messergebnisse des ersten Feuchtigkeitssensors und/oder des zweiten Feuchtigkeitssensors verwendet werden können.

In einer Variante sind der erste Feuchtigkeitssensor und der erste Temperatursensor außerhalb der Reinigungskammer in einer Abluftleitung angeordnet sind.

In einer weiteren Variante sind der erste Feuchtigkeitssensor und der erste Temperatursensor innerhalb der Reinigungskammer angeordnet.

In einer Variante sind der zweite Feuchtigkeitssensor und der zweite Temperatursensor außerhalb der Reinigungskammer in einer Zuluftleitung angeordnet sind. Diese Variante kann mit einer der vorstehenden Varianten kombiniert werden. Es ist also möglich, den ersten Feuchtigkeitssensor und den ersten Temperatursensor außerhalb der Reinigungskammer in einer Abluftleitung und den zweiten Feuchtigkeitssensor und den zweiten Temperatursensor ebenfalls außerhalb der Reinigungskammer, aber in einer Zuluftleitung anzuordnen. Ebenso ist es möglich, den ersten Feuchtigkeitssensor und den ersten Temperatursensor innerhalb der Reinigungskammer anzuordnen und den zweiten Feuchtigkeitssensor und den zweiten Temperatursensor außerhalb der Reinigungskammer in einer Zuluftleitung anzuordnen.

In einer Variante weist die Vorrichtung eine Abluftrückführleitung auf, die eine von der Reinigungskammer wegführende Abluftleitung mit einer zu der Reinigungskammer hinführenden Zuluftleitung verbindet. Auf diese Weise ist es möglich, einen Teil der im Betrieb der Vorrichtung aus der Reinigungskammer austretenden Abluft mit aus der Umgebung der Reinigungskammer stammenden Umgebungsluft zu vermischen. Durch diese Vermischung wird dann Zuluft gebildet, die der Reinigungskammer nachfolgend zugeführt wird.

Sämtliche Varianten und Ausgestaltungen des vorstehend beschriebenen Verfahrens sind in analoger Weise auf die vorstehend beschriebene Vorrichtung anwendbar, und umgekehrt. Dabei ist eine beliebige Kombination der erläuterten Varianten des Verfahrens und der Vorrichtung möglich.

Weitere Einzelheiten von Aspekten der der vorliegenden Erfindung werden anhand eines Ausführungsbeispiels und entsprechender Figuren näher erläutert. Es zeigen:

Figur 1    ein erstes Diagramm der absoluten Luftfeuchtigkeit in Abhängigkeit von der Zeit während einer Trocknungsphase eines Reinigungs- und Desinfektionsprozesses;

Figur 2    ein zweites Diagramm der absoluten Luftfeuchtigkeit in Abhängigkeit von der Zeit während einer Trocknungsphase eines Reinigungs- und Desinfektionsprozesses und

Figur 3    ein drittes Diagramm der absoluten Luftfeuchtigkeit in Abhängigkeit von der Zeit während einer Trocknungsphase eines Reinigungs- und Desinfektionsprozesses.

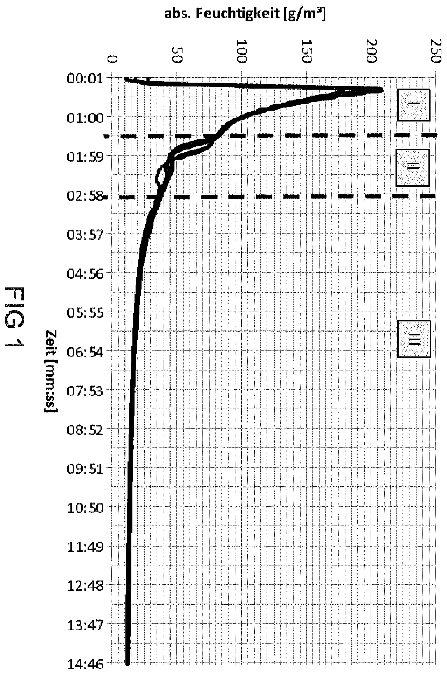

Die Figur 1 zeigt einen beispielhaften Verlauf der absoluten Feuchtigkeit der aus einer Reinigungskammer austretenden Abluft während der Trocknungsphase eines Reinigungs- und Desinfektionsprozesses von zahnmedizinischen Instrumenten und Geräten. Dabei sind die Feuchtigkeitskurven von fünf Wiederholversuchen übereinander gelagert.

Der Verlauf der absoluten Feuchtigkeit mit der Zeit lässt sich in drei Phasen gliedern, die in der Figur 1 mit I, II und III bezeichnet sind. In der Phase 1 beträgt die relative Luftfeuchtigkeit der Abluft in etwa 100 %. Durch eine Erhöhung der Temperatur der Abluft zu Beginn des Trocknungsprozesses steigt die absolute Feuchtigkeit an. Anschließend fällt sie jedoch ab, da nun eine tatsächliche Trocknung der Beladung einsetzt. Die Phase I dauert im Beispiel der Figur 1 rund 1,5 Minuten und geht dann in die Phase II über.

Der Feuchtigkeitsverlauf in der Phase II weist zwischen den einzelnen Wiederholversuchen sehr große Varianzen auf. Dies ist auf Messartefakte bei der Bestimmung der absoluten Feuchtigkeit zurückzuführen. Im Bereich von rund 3 Minuten nach Beginn der Trocknungsphase geht die Phase II in die Phase III über, in der die Feuchtigkeitswerte sämtlicher Experimente mit sehr geringer Varianz übereinanderliegen. Die absolute Feuchtigkeit der Abluft nähert sich dabei der absoluten Feuchtigkeit der Zuluft an. Ab einem Zeitpunkt von rund 10 Minuten nach Beginn der Trocknungsphase lässt sich praktisch keine weitere Reduzierung der absoluten Feuchtigkeit der Abluft mehr erreichen. Zu diesem Zeitpunkt beträgt Differenz zwischen der absoluten Feuchtigkeit der Abluft und der absoluten Feuchtigkeit der der Reinigungskammer zugeführte Zuluft nur noch 1 bis 5 g/m$^3$.

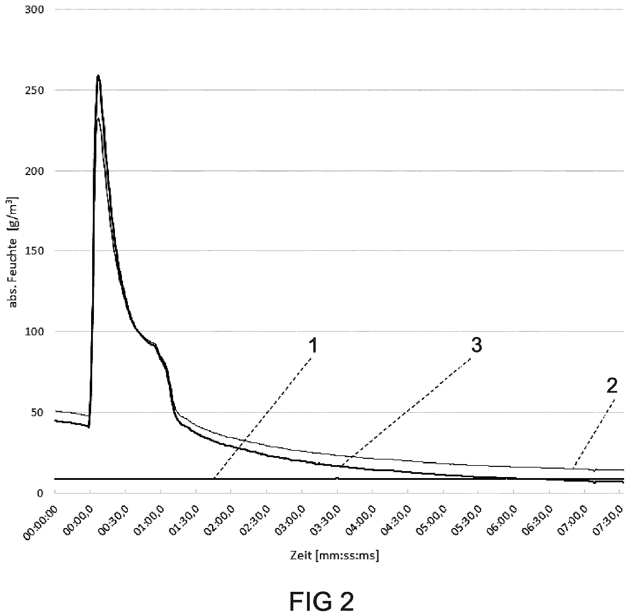

Den Verlauf der absoluten Feuchtigkeit kann man auch anhand des in der Figur 2 dargestellten Diagramms ersehen. So zeigt die Figur 2 den Verlauf der absoluten Feuchtigkeit 1 in der Zuluft, den Verlauf der absoluten Feuchtigkeit 2 in der Abluft und die Differenz 3 zwischen der absoluten Feuchtigkeit 2 in der Abluft und der absoluten Feuchtigkeit 1 in der Zuluft. Wie aus der Figur 2 gut ersichtlich ist, ändert sich die absolute Feuchtigkeit 1 in der Zuluft während der Trocknungsphase nur wenig. Demgegenüber nimmt die absolute Feuchtigkeit 2 in der Abluft während der Trocknungsphase ab und erreicht bei etwa 5,5 Minuten den Wert der absoluten Feuchtigkeit in der Zuluft. Nachfolgend ist keine weitere relevante Trocknung der in der Reinigungskammer des Reinigungs- und Desinfektionsgeräts enthaltenen Beladung möglich.

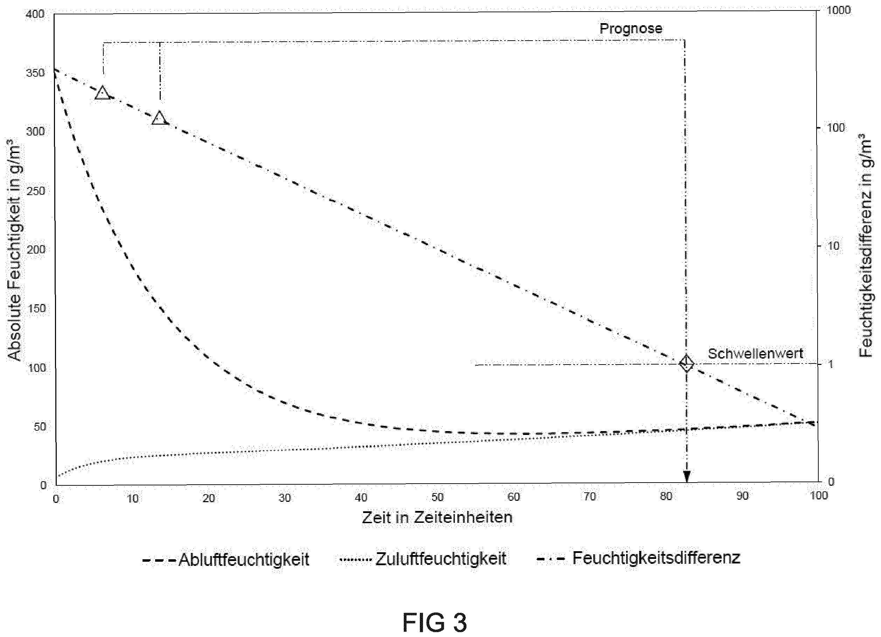

Die Figur 3 zeigt ein weiteres Diagramm des Feuchtigkeitsverlaufs während einer simulierten Trocknungsphase eines Verfahrens zur Reinigung und optionalen Desinfektion von Reinigungsgut. Die absolute Abluftfeuchtigkeit ist durch eine gestrichelte Kurve dargestellt. Die absolute Zuluftfeuchtigkeit ist durch eine gepunktete Kurve dargestellt. Die absolute Abluftfeuchtigkeit sinkt im Laufe der Trocknungsphase, während die Zuluftfeuchtigkeit leicht ansteigt. Dies ist darauf zurückzuführen, dass aus einem Gerät, in dem das Trocknungsverfahren durchgeführt wird, Abluft in einen Raum eingeleitet wird, in dem das Gerät aufgestellt wird. Aus diesem Raum wird aber auch die Zuluft in das Gerät gesaugt. Somit führt ein Einleiten der Abluft in die Umgebungsluft zu einem Anstieg der absoluten Feuchtigkeit in der Zuluft.

Die berechnete Feuchtigkeitsdifferenz ist in dem Diagramm der Figur 3 durch eine strichpunktierte Kurve dargestellt. Die Feuchtigkeitsdifferenz gibt die Differenz zwischen der absoluten Abluftfeuchtigkeit und der absoluten Zuluftfeuchtigkeit an. Diese Differenz wird zunächst zu zwei Zeitpunkten $t_i$ berechnet. Dazu wird die Zuluftfeuchtigkeit zum Zeitpunkt $t_i$ von der Abluftfeuchtigkeit zum selben Zeitpunkt $t_i$ abgezogen. Die entsprechend berechnete Differenz ist in der Figur 3 durch Dreiecke gekennzeichnet. Auf der Grundlage dieser beiden berechneten Differenzwerte wird dann eine Prognose des weiteren Verlaufs der Feuchtigkeitsdifferenz erstellt, so dass sich die strichpunktierte Kurve ergibt. Es kann dann prognostiziert werden, zu welchem Zeitpunkt ein vorgegebener Schwellenwert der Feuchtigkeitsdifferenz voraussichtlich erreicht wird.

Dieser vorgegebene Schwellenwert ist in dem Diagramm der Figur 3 durch eine Raute dargestellt. Die Prognose, die gemäß dem in der Figur 3 dargestellten Ausführungsbeispiel erstellt wurde, ergibt, dass der vorgegebene Schwellenwert bei rund 82 willkürlichen Zeiteinheiten erreicht wird. Bereits zu dem Zeitpunkt, an dem die zweite Differenz berechnet wird (also bei etwa 15 willkürlichen Zeiteinheiten), kann folglich ermittelt werden, wann ein prognostischer Abbruch der Trocknungsphase erfolgen kann, weil dann voraussichtlich das gewünschte Trocknungsergebnis erzielt wurde, also eine Differenz zwischen der Abluftfeuchtigkeit und der Zuluftfeuchtigkeit den vorgegebenen Schwellenwert erreicht bzw. unterschritten haben wird. Somit ist bereits zu einem frühen Zeitpunkt der Trocknungsphase absehbar, wie lange die Trocknungsphase voraussichtlich dauern wird.

**Patentansprüche**

1. Verfahren zur Steuerung der Dauer einer Trocknungsphase eines Verfahrens zur Reinigung und optionalen Desinfektion von Reinigungsgut, mit den folgenden Schritten:

   a) Messen eines relativen Feuchtigkeitswerts einer Abluftfeuchtigkeit (2) zu mindestens zwei voneinander verschiedenen Zeitpunkten $t_i$ unter Verwendung eines ersten Feuchtigkeitssensors,
   b) Messen der Temperatur von Abluft, welche aus einer Reinigungskammer, in der das Reinigungsgut getrocknet wird, ausströmt, mittels eines ersten Temperatursensors,
   c) Berechnen absoluter Feuchtigkeitswerte aus den gemessenen relativen Feuchtigkeitswerten und der gemessenen Temperatur, so dass die mindestens zwei Werte für die Abluftfeuchtigkeit (2) jeweils eine absolute Feuchtigkeit der Abluft angeben,
   d) Messen eines relativen Feuchtigkeitswerts einer Zuluftfeuchtigkeit (1) zu denselben Zeitpunkten $t_i$ unter Verwendung eines zweiten Feuchtigkeitssensors, wobei zusätzlich die Temperatur von Zuluft, welche zumindest teilweise aus einer Umgebung der Reinigungskammer angesaugt wird und zum Trocknen des Reinigungsguts verwendet wird, mittels eines zweiten Temperatursensors gemessen wird,
   e) Berechnen absoluter Feuchtigkeitswerte aus den gemessenen relativen Feuchtigkeitswerten und der gemessenen Temperatur, so dass die mindestens zwei Werte für die Zuluftfeuchtigkeit (1) jeweils eine absolute Feuchtigkeit der Zuluft angeben,
   f) Berechnen, auf der Grundlage der mindestens zwei Werte der absoluten Feuchtigkeit der Abluft und der mindestens zwei Werte der absoluten Feuchtigkeit der Zuluft, einer Zeitdauer, die voraussichtlich erforderlich ist, damit eine Differenz zwischen der absoluten Feuchtigkeit der Abluft und der absoluten Feuchtigkeit der Zuluft einen vorgebbaren Schwellenwert unterschreitet, und
   g) Beenden der Trocknungsphase, wenn die Zeitdauer verstrichen ist.

2. Verfahren nach Anspruch 1, **dadurch gekennzeichnet, dass** der vordefinierte Schwellenwert aus einem Bereich von 0,1 bis 20 g/m³ absoluter Feuchtigkeit ausgewählt ist.

3. Verfahren nach einem der vorherigen Ansprüche, **dadurch gekennzeichnet, dass** die Zeitdauer zwischen Schritt f) und Schritt g) unter Berücksichtigung eines weiteren Wertepaares der Abluftfeuchtigkeit (2) und der Zuluftfeuchtigkeit (1), das zu einem weiteren Zeitpunkt $t_i$ gemessen wurde, erneut berechnet wird und die zuvor berechnete Zeitdauer ersetzt.

4. Verfahren nach einem der Ansprüche 1 bis 3, **da-**

**durch gekennzeichnet, dass** die Zeitdauer gemäß folgender Formel berechnet wird:

$$t_{ges} = \log_a \frac{F_S}{b}$$

wobei

$$a = \left(\frac{\Delta F_{t_2}}{\Delta F_{t_1}}\right)^{\frac{1}{t_2 - t_1}}$$

$$b = \frac{\Delta F_{t_1}}{a^{t_1}}$$

$t_{ges}$ die Zeitdauer,
$F_S$ der Schwellenwert der Differenz zwischen der Abluftfeuchtigkeit (2) und der Zuluftfeuchtigkeit (1),
$t_1$ ein erster Messzeitpunkt während des Trocknungsprozesses,
t2 ein nach dem ersten Messzeitpunkt liegender zweiter Messzeitpunkt während des Trocknungsprozesses,
$\Delta F_{t1}$ die Differenz zwischen der Abluftfeuchtigkeit und der Zuluftfeuchtigkeit (1) zum Zeitpunkt $t_i = t_1$ und
$\Delta F_{t2}$ die Differenz zwischen der Abluftfeuchtigkeit und der Zuluftfeuchtigkeit (1) zum Zeitpunkt $t_i = t_2$ ist.

5.  Verfahren nach Anspruch 4, **dadurch gekennzeichnet, dass** der Schwellenwert in Abhängigkeit des Faktors a und/oder des Faktors b vorgegeben wird.

6.  Verfahren nach Anspruch 4 oder 5, **dadurch gekennzeichnet, dass** eine in der Reinigungskammer während der Trocknungsphase herrschende Trocknungstemperatur und/oder eine Drehzahl eines Gebläses, mit dem die Zuluft in die Reinigungskammer gefördert wird, in Abhängigkeit des Faktors a und/oder des Faktors b eingestellt werden.

7.  Verfahren nach einem der vorhergehenden Ansprüche, **dadurch gekennzeichnet, dass** die zur Berechnung der Zeitdauer herangezogenen Werte der Abluftfeuchtigkeit und/oder der Zuluftfeuchtigkeit (1) Mittelwerte aus gemessenen Werten der Abluftfeuchtigkeit und/oder der Zuluftfeuchtigkeit (1) sind.

8.  Verfahren nach einem der vorherigen Ansprüche, **dadurch gekennzeichnet, dass** ein Korrekturfaktor für einen Messwert der Abluftfeuchtigkeit (2) und/oder der Zuluftfeuchtigkeit (1) bestimmt und auf den entsprechenden Messwert angewandt wird, um einen korrigierten Messwert der Abluftfeuchtigkeit (2) und/oder der Zuluftfeuchtigkeit (1) zu erhalten.

9.  Vorrichtung zur Reinigung und optionalen Desinfektion von Reinigungsgut, mit einer Reinigungskammer zur Aufnahme von Reinigungsgut, einem ersten Feuchtigkeitssensor, einem zweiten Feuchtigkeitssensor, einem Steuergerät, einem ersten Temperatursensor, der zur Messung einer Temperatur von aus der Reinigungskammer austretender Abluft dient, und einem zweiten Temperatursensor, der zur Messung einer Temperatur von zumindest teilweise aus einer Umgebung der Reinigungskammer angesaugter Zuluft dient, wobei der erste Feuchtigkeitssensor zur Messung eines Feuchtigkeitswerts einer Abluftfeuchtigkeit (2) von aus der Reinigungskammer austretender Abluft dient, dass der zweite Feuchtigkeitssensor zur Messung eines Feuchtigkeitswerts einer Zuluftfeuchtigkeit (1) von zumindest teilweise aus einer Umgebung der Reinigungskammer angesaugter Zuluft dient,
    **dadurch gekennzeichnet,**
    **dass** das Steuergerät dafür vorgesehen und eingerichtet ist, ein Verfahren nach einem der vorhergehenden Ansprüche auszuführen.

10. Vorrichtung nach Anspruch 9, **dadurch gekennzeichnet, dass** der erste Feuchtigkeitssensor und der erste Temperatursensor außerhalb der Reinigungskammer in einer Abluftleitung angeordnet sind.

11. Vorrichtung nach Anspruch 9, **dadurch gekennzeichnet, dass** der erste Feuchtigkeitssensor und der erste Temperatursensor innerhalb der Reinigungskammer angeordnet sind.

12. Vorrichtung nach einem der Ansprüche 9 bis 11, **dadurch gekennzeichnet, dass** der zweite Feuchtigkeitssensor und der zweite Temperatursensor außerhalb der Reinigungskammer in einer Zuluftleitung angeordnet sind.

13. Vorrichtung nach einem der Ansprüche 9 bis 12, **dadurch gekennzeichnet, dass** die Vorrichtung eine Abluftrückführleitung aufweist, die eine von der Reinigungskammer wegführende Abluftleitung mit einer zu der Reinigungskammer hinführenden Zuluftleitung verbindet, damit ein Teil der im Betrieb der Vorrichtung aus der Reinigungskammer austretenden Abluft mit aus der Umgebung der Reinigungskammer stammenden Umgebungsluft vermischt werden kann, um Zuluft zu bilden, die der Reinigungskammer zugeführt wird.

14. Vorrichtung nach einem der Ansprüche 9 bis 13, **dadurch gekennzeichnet, dass** die Vorrichtung einen ersten Drucksensor, der zur Messung eines Luftdrucks innerhalb der Reinigungskammer oder von aus der Reinigungskammer austretender Abluft dient, und/oder einen zweiten Drucksensor, der zur Messung eines Luftdrucks von aus einer Umgebung der Reinigungskammer angesaugter Zuluft dient, aufweist.

**Claims**

1. A method of controlling the duration of a drying phase of a process for cleaning and optionally disinfecting items to be cleaned, comprising the following steps:

   a) measuring a relative humidity value of an exhaust air humidity (2) at at least two times $t_i$ different from each other by using a first humidity sensor,
   b) measuring the temperature of exhaust air that flows out of a cleaning chamber in which the items to be cleaned are dried, by means of a first temperature sensor,
   c) calculating absolute humidity values from the measured relative humidity values and the measured temperature so that the at least two values for the exhaust air humidity (2) each indicate an absolute humidity of the exhaust air,
   d) measuring a relative humidity value of a supply air humidity (1) at the same times $t_i$ by using a second humidity sensor, wherein in addition the temperature of supply air, which is at least partly sucked in from an environment of the cleaning chamber and is used for drying the items to be cleaned, is measured by means of a second temperature sensor,
   e) calculating absolute humidity values from the measured relative humidity values and the measured temperature so that the at least two values for the supply air humidity (1) each indicate an absolute humidity of the supply air,
   (f) calculating, on the basis of at least two values of the absolute humidity of the exhaust air and the at least two values of the absolute humidity of the supply air, a time period expected to be required for a difference between the absolute humidity of the exhaust air and the absolute humidity of the supply air to fall below a specifiable threshold value; and
   g) terminating the drying phase when the time period has elapsed.

2. The method according to claim 1, **characterized in that** the predefined threshold value is selected from a range of 0.1 to 20 g/m$^3$ of absolute humidity.

3. The method according to any of the previous claims, **characterized in that** the time period between step f) and step g) is again calculated by taking account of a further pair of values of the exhaust air humidity (2) and the supply air humidity (1), which has been measured at another time $t_i$ and which replaces the previously calculated time period.

4. The method according to any of claims 1 to 3, **characterized in that** the time period is calculated according to the following formula:

$$t_{total} = \log_a \frac{F_S}{b}$$

wherein

$$a = \left(\frac{\Delta F_{t_2}}{\Delta F_{t_1}}\right)^{\frac{1}{t_2 - t_1}}$$

$$b = \frac{\Delta F_{t_1}}{a^{t_1}}$$

$t_{total}$ is the time period,
$F_S$ is the threshold value of the difference between the exhaust air humidity (2) and the supply air humidity (1),
$t_1$ is a first time of measurement during the drying process,
$t_2$ is a second time of measurement during the drying process after the first time of measurement,
$\Delta F_{t1}$ is the difference between the exhaust air humidity and the supply air humidity (1) at the time $t_i = t_1$ and
$\Delta F_{t2}$ is the difference between the exhaust air humidity and the supply air humidity (1) at the time $t_i = t_2$.

5. The method according to claim 4, **characterized in that** the threshold value is specified as a function of the factor a and/or the factor b.

6. The method according to claim 4 or 5, **characterized in that** a drying temperature existing in the cleaning chamber during the drying phase and/or a rotational speed of a blower by means of which the supply air is conveyed into the cleaning chamber are set as a function of the factor a and/or the factor b.

7. The method according to any of the preceding claims, **characterized in that** the values of the exhaust air humidity and/or of the supply air humidity (1) used for calculating the time period are mean values from measurement values of the exhaust air humidity and/or the supply air humidity (1).

**8.** The method according to any of the previous claims, **characterized in that** a correction factor for a measurement value of the exhaust air humidity (2) and/or the supply air humidity (1) is determined and applied to the corresponding measurement value in order to obtain a corrected measurement value of the exhaust air humidity (2) and/or the supply air humidity (1).

**9.** A device for cleaning and optionally disinfecting items to be cleaned, comprising a cleaning chamber for receiving items to be cleaned, a first humidity sensor, a second humidity sensor, a control unit, a first temperature sensor that is used for measuring a temperature of exhaust air exiting from the cleaning chamber, and a second temperature sensor that is used for measuring a temperature of supply air at least partly sucked in from an environment of the cleaning chamber, wherein the first humidity sensor is used for measuring a humidity value of an exhaust air humidity (2) of exhaust air exiting from the cleaning chamber, and the second humidity sensor is used for measuring a humidity value of a supply air humidity (1) of supply air at least partly sucked in from an environment of the cleaning chamber, **characterized in that** the control unit is intended and equipped to carry out a method according to any of the preceding claims.

**10.** The device according to claim 9, **characterized in that** the first humidity sensor and the first temperature sensor are arranged outside the cleaning chamber in an exhaust air conduit.

**11.** The device according to claim 9, **characterized in that** the first humidity sensor and the first temperature sensor are arranged within the cleaning chamber.

**12.** The device according to any of claims 9 to 11, **characterized in that** the second humidity sensor and the second temperature sensor are arranged outside the cleaning chamber in a supply air conduit.

**13.** The device according to any of claims 9 to 12, **characterized in that** the device comprises an exhaust air return conduit that connects an exhaust air conduit leading away from the cleaning chamber to a supply air conduit leading towards the cleaning chamber, so that part of the exhaust air exiting from the cleaning chamber during operation of the device can be mixed with ambient air originating from the environment of the cleaning chamber to form supply air that is supplied to the cleaning chamber.

**14.** The device according to any of claims 9 to 13, **characterized in that** the device comprises a first pressure sensor that is used for measuring an air pressure inside the cleaning chamber or for measuring exhaust air exiting from the cleaning chamber and/or a second pressure sensor that is used for measuring an air pressure of supply air sucked in from an environment of the cleaning chamber.

**Revendications**

**1.** Procédé de commande de la durée d'une phase de séchage d'un processus de nettoyage et éventuellement de désinfection d'objets à nettoyer, comprenant les étapes suivantes :

a) mesurer une valeur d'humidité relative d'une humidité d'air d'échappement (2) à au moins deux moments différents $t_i$ en utilisant un premier capteur d'humidité,
b) mesurer la température de l'air d'échappement sortant d'une chambre de nettoyage dans laquelle les objets à nettoyer sont séchés, au moyen d'un premier capteur de température,
c) calculer des valeurs d'humidité absolues à partir des valeurs d'humidité relatives mesurées et de la température mesurée, de sorte que les au moins deux valeurs pour l'humidité d'air d'échappement (2) indiquent chacune une humidité absolue de l'air d'échappement,
d) mesurer une valeur d'humidité relative d'une humidité d'air d'alimentation (1) aux mêmes moments $t_i$ en utilisant un deuxième capteur d'humidité, dans lequel en outre la température de l'air d'alimentation qui est au moins partiellement aspiré à partir d'un environnement de la chambre de nettoyage et qui est utilisé pour sécher les objets à nettoyer est mesurée au moyen d'un deuxième capteur de température,
e) calculer des valeurs d'humidité absolues à partir des valeurs d'humidité relatives mesurées et de la température mesurée, de sorte que les au moins deux valeurs pour l'humidité d'air d'alimentation (1) indiquent chacune une humidité absolue de l'air d'alimentation,
f) calculer, sur la base des au moins deux valeurs de l'humidité absolue de l'air d'échappement et des au moins deux valeurs de l'humidité absolue de l'air d'alimentation, une période de temps attendue pour qu'une différence entre l'humidité absolue de l'air d'échappement et l'humidité absolue de l'air d'alimentation tombe en dessous d'une valeur seuil prédéterminable, et
g) terminer la phase de séchage lorsque la période de temps s'est écoulée.

**2.** Procédé selon la revendication 1, **caractérisé en ce que** la valeur seuil prédéfinie est choisie dans une gamme de 0,1 à 20 g/m³ d'humidité absolue.

**3.** Procédé selon l'une quelconque des revendications précédentes, **caractérisé en ce que** la période de temps entre l'étape f) et l'étape g) est calculée de nouveau en tenant compte d'une autre paire de valeurs de l'humidité de l'air d'échappement (2) et de l'humidité de l'air d'alimentation (1), qui ont été mesurées à un autre moment $t_i$, et remplace la période de temps précédemment calculée.

**4.** Procédé selon l'une quelconque des revendications 1 à 3, **caractérisé en ce que** la période de temps est calculée selon la formule suivante :

$$t_{total} = log_a \frac{F_S}{b}$$

où

$$a = \left(\frac{\Delta F_{t_2}}{\Delta F_{t_1}}\right)^{\frac{1}{t_2-t_1}}$$

$$b = \frac{\Delta F_{t_1}}{a^{t_1}}$$

$t_{total}$ est la période de temps,
$F_S$ est la valeur seuil de la différence entre l'humidité de l'air d'échappement (2) et l'humidité de l'air d'alimentation (1),
$t_1$ est un premier instant de mesure pendant le processus de séchage,
$t_2$ est un deuxième instant de mesure pendant le processus de séchage après le premier instant de mesure,
$\Delta F_{t_1}$ est la différence entre l'humidité de l'air d'échappement et l'humidité de l'air d'alimentation (1) au moment $t_i = t_1$ et
$\Delta F_{t_2}$ est la différence entre l'humidité de l'air d'échappement et l'humidité de l'air d'alimentation (1) au moment $t_i = t_2$.

**5.** Procédé selon la revendication 4, **caractérisé en ce que** la valeur seuil est prédéterminée en fonction du facteur a et/ou du facteur b.

**6.** Procédé selon la revendication 4 ou 5, **caractérisé en ce qu'**une température de séchage régnant dans la chambre de nettoyage pendant la phase de séchage et/ou une vitesse de rotation d'un ventilateur avec lequel l'air d'alimentation est transporté dans la chambre de nettoyage sont réglées en fonction du facteur a et/ou du facteur b.

**7.** Procédé selon l'une quelconque des revendications précédentes, **caractérisé en ce que** les valeurs de l'humidité de l'air d'échappement et/ou de l'humidité de l'air d'alimentation (1) utilisées pour calculer la durée sont des valeurs moyennes issues de valeurs mesurées de l'humidité de l'air d'échappement et/ou de l'humidité de l'air d'alimentation (1).

**8.** Procédé selon l'une quelconque des revendications précédentes, **caractérisé en ce qu'**un facteur de correction pour une valeur de mesure de l'humidité de l'air d'échappement (2) et/ou de l'humidité de l'air d'alimentation (1) est déterminé et appliqué à la valeur de mesure correspondante pour obtenir une valeur de mesure corrigée de l'humidité de l'air d'échappement (2) et/ou de l'humidité de l'air d'alimentation (1).

**9.** Appareil de nettoyage et éventuellement de désinfection d'objets à nettoyer, comprenant une chambre de nettoyage destinée à recevoir des objets à nettoyer, un premier capteur d'humidité, un deuxième capteur d'humidité, un dispositif de commande, un premier capteur de température qui sert à mesurer une température de l'air d'échappement sortant de la chambre de nettoyage, et un deuxième capteur de température qui sert à mesurer une température de l'air d'alimentation aspiré au moins partiellement d'un environnement de la chambre de nettoyage, le premier capteur d'humidité servant à mesurer une valeur d'humidité d'une humidité d'air d'échappement (2) de l'air d'échappement sortant de la chambre de nettoyage, et le deuxième capteur d'humidité sert à mesurer une valeur d'humidité d'une humidité d'air d'alimentation (1) de l'air d'alimentation aspiré au moins partiellement d'un environnement de la chambre de nettoyage,
**caractérisé en ce que**
le dispositif de commande est prévu et configuré pour la mise en œuvre d'un procédé selon l'une quelconque des revendications précédentes.

**10.** Appareil selon la revendication 9, **caractérisé en ce que** le premier capteur d'humidité et le premier capteur de température sont agencés à l'extérieur de la chambre de nettoyage dans un conduit d'évacuation d'air.

**11.** Appareil selon la revendication 9, **caractérisé en ce que** le premier capteur d'humidité et le premier capteur de température sont agencés à l'intérieur de la chambre de nettoyage.

**12.** Appareil selon l'une quelconque des revendications 9 à 11, **caractérisé en ce que** le deuxième capteur d'humidité et le deuxième capteur de température sont agencés à l'extérieur de la chambre de nettoyage dans un conduit d'alimentation d'air.

**13.** Appareil selon l'une quelconque des revendications 9 à 12, **caractérisé en ce que** l'appareil comprend un conduit de retour d'air d'échappement reliant un conduit d'échappement d'air s'éloignant de la chambre de nettoyage à un conduit d'alimentation d'air se dirigeant vers la chambre de nettoyage, pour permettre à une partie de l'air d'échappement sortant de la chambre de nettoyage pendant le fonctionnement de l'appareil d'être mélangée avec de l'air ambiant provenant de l'environnement de la chambre de nettoyage pour former de l'air d'alimentation qui est amené à la chambre de nettoyage.

**14.** Appareil selon l'une quelconque des revendications 9 à 13, **caractérisé en ce que** l'appareil comprend un premier capteur de pression, qui sert à mesurer une pression d'air à l'intérieur de la chambre de nettoyage ou à mesurer de l'air d'échappement sortant de la chambre de nettoyage, et/ou un deuxième capteur de pression, qui sert à mesurer une pression d'air de l'air d'alimentation aspiré depuis un environnement de la chambre de nettoyage.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1689280 B1
- DE 102008043176 A1
- DE 102013106775 A1
- EP 0953315 A1
- WO 2004019750 A1
- CN 105105694 A1
- JP 2011200392 A
- DE 102011083179 A1